Europäisches Patentamt
European Patent Office
Office européen des brevets

(19)

(11) Publication number: 0 222 310 A2

(12)

# EUROPEAN PATENT APPLICATION

(21) Application number: 86115321.1

(22) Date of filing: 05.11.86

(51) Int. Cl.[4]: G 01 N 33/569
C 12 Q 1/68
//A61K39/21

(30) Priority: 13.11.85 US 797493

(43) Date of publication of application: 20.05.87 Bulletin 87/21

(84) Designated Contracting States: AT BE CH DE ES FR GB GR IT LI LU NL SE

(71) Applicant: THE WISTAR INSTITUTE
36th Street at Spruce
Philadelphia Pennsylvania 19104(US)

(72) Inventor: Koprowski, Hilary
334 Wynnewood Road
Wynnewood, PA 19096(US)

(72) Inventor: De Freitas, Elaine C.
731 Newtown Road
Villanova PA.19095(US)

(72) Inventor: Gallo, Robert C.
8513 Thornden Terrace
Bethesda, MD 20834(US)

(74) Representative: Patentanwälte Wenzel & Kalkoff
Flasskuhle 6 Postfach 2448
D-5810 Witten(DE)

(54) **Methods for diagnosis and therapy of multiple sclerosis.**

(57) Methods of diagnosis are taught for multiple sclerosis. A viral agent is implicated in the disease and methods for identifying the virus and viral-infected cells are disclosed. Vaccines comprising viral proteins are presented. The new virus is in the human retrovirus family known to cause both adult T-cell leukaemia and acquired immune deficiency syndrome.

EP 0 222 310 A2

# METHODS FOR DIAGNOSIS AND THERAPY OF MULTIPLE SCLEROSIS

The invention described herein was made in the course of work under grants from the National Institutes of Health.

## BACKGROUND OF THE INVENTION

Multiple sclerosis (MS) is a demyelinating disease of the central nervous system. Some features of the disease are shared by autoimmune diseases. For example, increases in absolute numbers of T-cells in the cerebrospinal fluid (CSF) and disturbances in the relative proportions of T-cell subpopulations during exacerbation or remission of the disease are characteristic of both MS and autoimmune diseases. A high level of oligoclonal immunoglobulin in CSF of MS patients is often found.

Further, there are common characteristics shared by autoimmune diseases and human retrovirus infections. The human T-cell is the main target of all known human retroviruses. T4-positive (helper-inducer) lymphocytes are transformed by human T-cell lymphotropic virus HTLV-I and -II and are killed by HTLV-III, resulting in T-cell subpopulation imbalances. Occasionally, polyclonal B-cell activation and immunoglobulin production has been associated with human retrovirus infections.

The cause of multiple sclerosis is not known. It has been postulated that a viral infection plays a part in the disease but there is no formal proof that any virus is involved in the aetiology of the disease.

DESCRIPTION OF THE FIGURES

Figure 1a shows the relative amounts of antibodies reactive with HTLV-I p24 gag protein and AMV p27 protein in cerebrospinal fluid (CSF) from Swedish MS patients, patients with other neurological diseases, and normal donors.

Figure 1b shows the relative amounts of antibodies reactive with HTLV-I p24 gag protein in sera of Swedish MS patients and normal Swedish donors.

Figure 2 shows a comparison of the reactivity of serum antibodies of MS patients, other neurological disease patients (OND), and normal blood donors towards HTLV-I, -II, and -III.

Figure 3 shows the antibody reactivity with HTLV-I p24 gag protein in serial samples of CSF in sera obtained from MS patients.

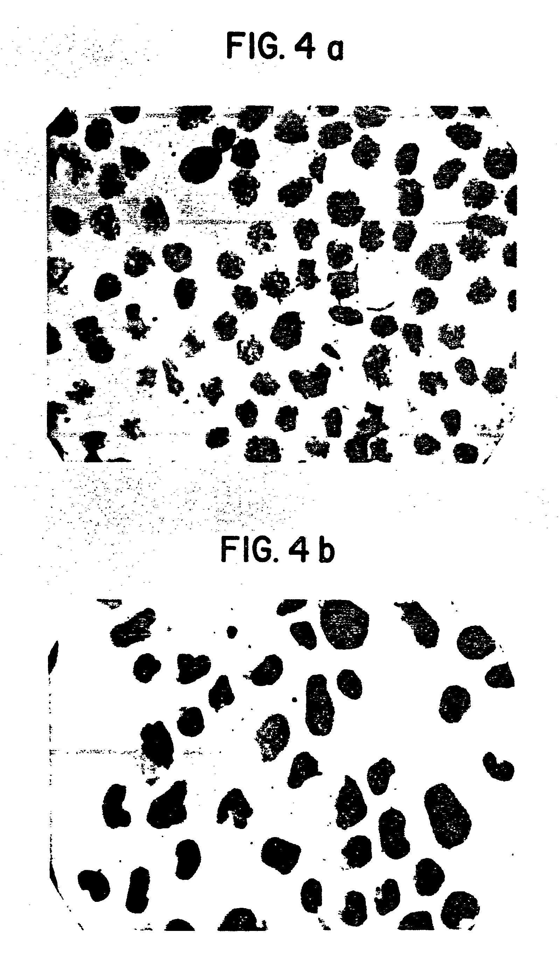

Figure 4 shows that cells can be detected in cultured cells from CSF of MS patients which epxress RNA with homology to HTLV-I.

SUMMARY OF THE INVENTION

It is an object of the invention to provide diagnostic methods of identify human patients suffering from multiple sclerosis.

It is another object of the present invention to provide methods of identifying cells which are infected by HTLV-IV.

It is an object of the present invention to provide a method for identifying HTLV-IV virus.

It is a further object of the present invention to provide vaccines for immunizing humans against HTLV-IV virus.

In accordance with this invention, it has been discovered that HTLV-IV virus is associated with multiple sclerosis and a test for the presence of HTLV-IV virus in CSF or serum can be used as the basis of a diagnostic method for directly testing human patients for multiple sclerosis. For example, CSF or serum can be tested for the presence of antibodies which bind to HTLV-IV proteins. Alternatively, antibodies can be raised which are reactive with the HTLV-IV proteins, and those antibodies can be used to test human body fluids for the presence of the HTLV-IV virus directly.

Further, in accordance with this invention a method is provided for identifying an HTLV-IV virus comprising: hybridizing viral RNA with a nucleic acid probe comprising HTLV-IV homologous sequences and selecting HTLV-IV RNA that hybridizes to said probe. Still other aspects of the invention, including methods of identifying infected cells, are described in the specification.

DETAILED DESCRIPTION OF THE INVENTION

Applicants have found a new virus in the family of human retroviruses which is designated HTLV-IV. The virus is found in the cerebrospinal fluid cells of patients having multiple sclerosis. Cells from other tissues may also be infected. HTLV-IV is different from all known human T-cell lymphotropic viruses, however, it shares more protein homology with HTLV-I than with either HTLV-II or -III. HTLV-I is the causative agent in the syndrome called adult T-cell leukaemia (ATL).

A number of findings has led to the discovery of HTLV-IV. Many MS patients were found to have antibody in either serum or cerebrospinal fluid which cross-reacted with HTLV-I, and yet the antibody did not behave in the same manner as antibody from ATL patients. In addition cells isolated from the cerebrospinal fluid of MS patients were found to express RNA which cross-hybridized with RNA derived from HTLV-I, and yet the cross-hybridization was not detected under conditions which would detect RNA in cells from ATL patients. Thus, a virus similar but not identical to HTLV-I is present in these MS patients, which does not cause the same symptoms as ATL.

The present invention contemplates a method of identifying a cell which is infected by HTLV-IV, wherein in situ hybridization at low stringency conditions with a nucleic acid probe comprising sequences of HTLV-I is used to identify an infected cell. Applicants have found that HTLV-IV RNA can be found in cells from MS patients using RNA hybridization probes. If hybridization is performed under conditions where totally homologous sequences are detected herein termed high

stringency conditions, for example, at 50°C in 50% formamide and 2 x SSC, HTLV-IV cannot be detected. However, if the hybridization conditions are made less stringent (herein termed low stringency), by reducing the temperature to 45°C (where sequences with up to about 10% more mismatch can be detected than at high stringency), HTLV-IV sequences can be detected in approximately $1 \times 10^{-4}$ to $1 \times 10^{-5}$ of the cells examined. Functionally equivalent high and low stringency conditions can be obtained, as is well known in the art, at other temperatures, depending on the monovalent salt and formamide concentrations in the particular hybridization medium used. Many hybridization media are known. Choice of hybridization medium and temperature are well within the skill of the art. Although no hybridization has been detected at high stringency conditions to HTLV-I probes, it is possible that some infected cells make more RNA than those cells already examined. In that case, some hybridization may occur at high stringency, however, the difference in amount of hybridization occurring at low and high stringency conditions will be significant.

Such a hybridization assay can also be performed on viral RNA extracted from virus particles. Means of extraction of viral RNA are well known in the art, and include protease treatment, phenol extraction and the like. Such a hybridization assay can also be performed on DNA isolated from infected cells, for examples, using the well-known Southern hybridization technique. (Journal of Molecular Biology <u>98</u>, 503, 1975.)

Hybridization probes can be derived from HTLV-I. In particular, the sequences from the LTR or p24 <u>gag</u> coding regions are known to be suitable. (Seiki, et al. Proc. Natl. Acad. Sci. USA <u>80</u>, 3618-3622, (1983) and Josephs, et al. Virology <u>139</u>, 340-345 (1984)). Probes can be labeled with any suitable radioactive atom, including but not limited to $^{14}C$, $^{32}P$, or $^{35}S$. Applicants have found the use of high specific activity $^{35}S$-containing nucleotides particularly sensitive. Alternate non-isotopic means of labeling are also suitable, such as flourescence probes. Nucleic acid probes may consist of either RNA or DNA. HTLV-IV is not detectable under high or low stringency conditions using hybridization probes derived from the genome of HTLV-III virus.

The localization of the RNA hybridization to particular cells desirably is accomplished by means of autoradiography. Because the hybridizing cells are rare ($10^{-4}$ cells) and each cell makes only a small amount of RNA, every attempt should be made to maintain sensitivity of the assay conditions. A means of reducing background exposure to maintain sensitivity is to digest non-hybridized probe with enzymes specific for single stranded nucleic acids, e.g., RNase $T_1$, and RNase A. This, in addition to extensive washing with the hybridization medium (without probe), lowers the background, enhancing the sensitivity of signal detection.

Patients who are infected with HTLV-IV virus can be diagnosed employing any techniques for the identification of a virus infection known in the art. One such technique is based on production of antibodies by the patient that are immunoreactive with the virus. These antibodies are also able to immunoreact with certain proteins of other human retroviruses. More specifically, it is possible to detect HTLV-IV antibodies by screening a body fluid selected from the group consisting of CSF and serum for immunological cross-reactivity with the p24 protein of HTLV-I. Lower levels of antibody reactivity against p24 of HTLV-I are found in MS patients than is found in serum from an HTLV-I leukaemic patient.

In order to distinguish the p24 antibody binding found in body fluids of HTLV-I and HTLV-IV infected patients, competitive binding assays can be performed. In such a competitive assay the antibodies in the serum or CSF are first reacted with the inhibiting substance, in this case disrupted virions of HTLV-I. Then p24 protein of HTLV-I can be added to the serum and the amount of p24 binding can be determined.

The amount of antibody binding to p24 protein can be determined by any of a variety of techniques known in the art. Applicants have found RIA (radioimmunoassay) and ELISA (enzyme-linked immuno-absorbent assay) particularly useful. In RIA, the p24 protein is radiolabeled, for example, with $^{125}I$. The amount of label which binds to the antibodies can be easily quantitated with a gamma radiation counter. In ELISA, the serum is contacted with immobilized p24 antigen. The amount of antibody in the serum which binds to the immobilized antigen can be quantitated by contacting with, for example, a goat anti-human IgG conjugated to peroxidase. Using a simple colorimetric enzyme assay for peroxidase, the amount of antibody binding to the immobilized p24 antigen can be determined. Other means of quantitating antibody-antigen reactions are well known in the art and can be used in the practice of this invention.

In competitive binding assays with serum from HTLV-I leukaemic patients, HLTV-I disrupted virions completely compete for the anti-p24 antibody binding in the serum. However, in serum from MS patients, the HTLV-I disrupted virions do not completely inhibit the p24 protein binding to serum antibodies. Thus, antibodies in serum from patients can be distinguished between those containing anti-HTLV-I and anti-HTLV-IV antibodies. Applicants have found that in such an assay, complete inhibition is generally attainable at levels of greater than about 85% inhibition. Incomplete inhibition is generally less than about 80% inhibition.

Other means of screening serum for HTLV-IV antibodies include screening for antibodies which react with HTLV-IV proteins, or HTLV-I proteins other than p24 gag protein. Methods of detecting antibody-antigen reactions are those already discussed above.

Once patients have been found who test positive for HTLV-IV infection, the HTLV-IV virus can be located in particular cells of body specimens and isolated. Body specimens may be taken from any tissue or fluid suspected of containing HTLV-IV infected cells. Serum and CSF are convenient sources of cells, although biopsy samples may also contain infected cells. Particular cells expressing viral RNA can be detected by in situ hybridization with nucleic acid probes which are homologous to the HTLV-IV genome. Such probes can be derived from HTLV-I (for example, the LTR or p24 gag genes) or from the HTLV-IV genome. Infected cells can also be detected by in situ antibody binding. For example, using antibody immunoreactive with HTLV-IV proteins or HTLV-I p24 gag protein, cells can be detected which express HTLV-IV proteins.

Isolated HTLV-IV virus can be propagated by growing on permissive cells in culture. Cell lines can be screened for permissiveness by coculturing with a source of virus. Evidence of virus growth can be detected by assaying for reverse transcriptase or other antigens identified with the virus. Virus preparations can be used inter alia to inoculate mammals such as rabbits or goats to generate polyclonal serum containing antibodies directed against HTLV-IV. In addition, monoclonal antibodies can be readily generated by inoculating mice, for example, with virus preparations and later fusing splenocytes from the immunized mice with myeloma cells to generate hybridoma cell lines which produce antibodies directed against HTLV-IV proteins. Such antibody producing methods are well known in the art.

Patients who are infected with HTLV-IV virus can be diagnosed employing any technique for the identification of a virus infection known in the art. For example, HTLV-IV proteins may be used in a diagnostic method to screen patients directly for antibodies which react with the proteins. As in all the immunological methods of the present invention, antibodies may be found in either serum or CSF. Proteins may be purified or may consist simply of disrupted or whole virions. Disrupted virions are typically preparations containing a non-ionic detergent, such as Triton X-100.

In another method for diagnosing HTLV-IV infection, antibodies immunoreactive with HTLV-IV proteins generated as discussed above, either polyclonal or monoclonal, can be used to screen human patients suffering from multiple sclerosis in a simple diagnostic method. A body specimen of a human patient can be contacted with the antibodies discussed above, under conditions where antibody-antigen complexes form and are stable. By determining the presence or absence of antibody antigen complexes, a simple diagnosis can be made as to the presence of the HTLV-IV virus. Presence or absence of antibody-antigen complexes can be determined by any of the techniques known in the art, including immunodiffusion, RIA, ELISA and the like. Presence of HTLV-IV virus indicates multiple sclerosis, or perhaps a subtype of multiple sclerosis.

Once the HTLV-IV virus is isolated, and permissive cell lines identified, vaccines for immunizing humans against the multiple sclerosis can be prepared. Vaccines comprise protein encoded by the viral nucleic acid isolated from a cell infected with HTLV-IV. The HTLV-IV protein may be a particular, isolated and purified protein or may be a mixture of viral proteins. A typical purification procedure for p24 gag protein of HTLV-I is described in Journal of Virology, Vol. 38, pp. 906-915 (1981). Similar methods can be used for isolating structural protein of HTLV-IV. A preparation of attenuated virions can also be used to raise antibodies in the immunized human. Attenuation can be achieved by any means known in the art including physical or chemical treatment such as formalin treatment. Preferably the RNA in such a preparation is rendered non-infective by chemical or enzymatic treatment. Whole virus can be used to immunize humans if the virus is a variant that is no longer pathogenic.

The following examples are meant as exemplification only and are not intended to limit the scope of the invention.

Example 1

This example demonstrates that antibodies are present in the CSF and serum of MS patients which are reactive with HTLV-I p24 gag protein.

CSF samples were collected from Swedish MS patients, patients with other neurological diseases (OND) and normal donors. All samples were tested simultaneously in the same laboratory. The 35 Swedish patients were diagnosed to have MS based on the criteria of McDonald and Halliday (British Medical Bulletin, 33, 4-9 (1977)). These patients were between 19 and 57 years of age when the samples were obtained. Fourteen were male and 21 were female. The disease duration ranged from 10 days to 21 years from initial onset of symptoms. In 15 patients, optic neuritis marked clinical onset of the disease. Four patients were incapacitated, 11 had symptoms, and 20 were able to carry on normal activities. Samples from 15 patients were obtained during ongoing exacerbation, which had started between 5 days and 3 months before sampling. Fourteen patients were in remission. Six patients had progressive disease. One patient was receiving adrenocorticotrophin (ACTH) therapy at the time of sampling and two patients had received adrenocorticotrophin five and six months, respectively, before sampling.

In addition, a single CSF sample was obtained from each of 18 patients with neurological diseases other than MS (OND) and from 7 healthy subjects. For control purposes, serum samples were also obtained from 80 healthy Swedish hospital staff members and from blood donors.

Single CSF samples from each of 35 MS patients were evaluated for the presence of antibody reacting with the major core protein, p24 of HTLV-I (Kalynaraman et al. Journal of Virology Vol. 81, 906-915 (1981)) by ELISA. (See, for example, Saxinger, et al. Lab Investigation, 49, 371-377 (1983).) A single CSF sample from each patient was tested using 2 ug/ml p24 or p27 bound to Immulon II plates (Dynatech, Alexandria, Virginia) in carbonate-bicarbonate buffer (pH 9.9). Nonspecific binding was blocked by addition of 1% bovine serum albumin in phosphate buffered saline. All samples were diluted so that the concentration of IgG equalled that found in normal CSF, then applied to the plates. After one hour at 23°C, an optimal concentration of peroxidase-conjugated goat anti-human IgG (heavy and light chain supplied by KPL, Gaithersburg, Maryland) was added for one hour at 23°C. After extensive washing the plates were incubated with 2,2-azino-di-(3-ethylmenzthizaline) sulfonate and hydrogen peroxide and the plates read at 405 nm in a Titertech Multiscan (El Fab, Helsinki, Finland). Assay conditions were standardized using HTLV-I and HTLV-III positive sera and normal control sera. Each assay included serum samples from an HTLV-I leukaemic patient (F0920) as a positive control and NIH HTLV-I negative sera as a negative control.

Data analysis by the Mann-Whitney U-test indicated that the MS CSF samples differed significantly from all OND CSF samples ($p = 0.059$) and from OND samples taken from patients with an intact blood brain barrier ($p = 0.002$). The results in Figure 1a show that the CSF from MS patients had higher levels of antibodies reactive with the HTLV-I p24 than CSF from normal individuals and most of the OND patients. The three OND CSF samples that were notably reactive with p24 were from a Guillian-Barre patient, a patient with meningitis of unknown origin, and a patient with lymphocytic meningoradiculitis. None of the 35 MS CSF samples showed reactivity with the p27 of avian myeloblastosis virus (AMV) which has low sequence homology with HTLV-I p24, demonstrating the specificity of the antibodies detected by HTLV-I p24. Of the 18 OND samples tested, only the CSF from the meningoradiculitis patient was reactive with AMV p27; that sample was also reactive with HTLV-I p24.

In Figure 1b 35 serum samples from Swedish MS patients and 43 normal Swedish donors were screened for antibodies reactive with HTLV-I p24 gag protein. Serum samples were diluted 1:150 in phosphate buffered saline containing 1% bovine serum albumin, and assayed for IgG antibody against HTLV-I p24 by ELISA as described above.

Data analysis by the Mann-Whitney U-test indicated that the MS sera differed significantly from the normal sera (p is less than 0.001). The mean absorbance for the MS patient sera was 0.746 ± 0.155, whereas the mean in the normal sera was 0.325 ± 0.102. In contrast, the HTLV-I leukaemic sera F0920 routinely showed reactivity at an absorbance of 1.55 - 1.57 in the same assay at the same serum dilution.

Example 2

This example demonstrates the specificity of the anti-p24 HTLV-I antibody found in the serum of MS patients.

For the RIA (radioimmunoassay) patients' sera were diluted 1:25 and incubated overnight on plates precoated with goat anti-human IgG as a "catcher" antibody. Inhibition of HTLV-I p24 antibody was determined by reacting various concentrations of disrupted virions of HTLV-I and -III, AMV p27, and HTLV-I p24 with wells containing patients' sera for three hours at 37°C. After washing to remove inhibitor, binding of HTLV-I p24 was assessed by incubating all wells with 0.25 ug of $^{125}$I-labeled HTLV-I p24 (1 x $10^5$ cpm/well). Percent inhibition is expressed as 100 x (cpm bound with no competing antigen - cpm bound with competing antigen)/cpm bound with no competing antigen. The values are the percentage inhibition of $^{125}$I-p24 binding by 0.5 ugs of competing antigen.

Conditions to effectively compete anti-HTLV-I p24 antibody with disrupted virions of HTLV-I and HTLV-III were established using HTLV-I and HTLV-III positive sera. Data are shown in Table 1 below.

For the ELISA (enzyme linked immunoabsorbent assay) equal volumes of patients' sera (diluted 1:100) were incubated with soluble antigent (0.7 ug/ml) for 30 minutes, then applied to plates pre-coated with HTLV p24 and the determination of binding performed as described in Example 1. Percent inhibition was calculated as 100 x ($A_{405}$ of serum without inhibitor - $A_{405}$ of serum with inhibitor)/$A_{405}$ of serum without inhibitor.

TABLE 1

Competitive Inhibition for HTLV-I p24 Antibodies
in Sera of Swedish MS Patients

%Inhibition of HTLV-I p24 Antibody

| | Competing Antigen | | | |
|---|---|---|---|---|
| | Disrupted Virions | | HTLV-I | AMV |
| Patient | HTLV-I | HTLV-III | p24 | p27 |
| 13-10+ | 46 | 44 | 86 | 6 |
| 01-12+ | 29 | 30 | 83 | 4 |
| 3380* | 58 | 51 | 98 | NT |
| 2268* | 40 | 31 | 83 | -8 |
| 2167* | 61 | 29 | 89 | NT |
| 1738* | 29 | 15 | 86 | NT |
| F0920*† | 90 | 28 | 94 | NT |

\+ RIA

* ELISA

† Serum from an HTLV-I leukaemic patient was diluted 1:500 and inhibitors used at 0.35 ug/ml.

NT means not tested.

The data show that in competitive inhibition in radioimmunoassay and enzyme linked immuno-asborbant assay from serum samples from 6 MS patients, anti-HTLV-I p24 antibodies were completely competed with p24 HTLV-I and partially competed with disrupted HTLV-I and HTLV-III virions, but not with AMV p24. This was in contrast to results observed with a control serum from an HTLV-I leukaemic patient wherein HTLV-I anti-p24 antibody was completely inhibited by HTLV-I virions and HTLV-I p24, but not by HTLV-III virions.

Example 3

This example demonstrates the reactivity in ELISA of HTLV-I, HTLV-II, and HTLV-III disrupted virions with serum antibodies from 35 MS patients, 10 OND patients and 80 normal Swedish blood donors.

The results of such assays, performed as described in Example 1, are shown in Figure 2, panels a, b, and c. The data is expressed in terms of an ELISA ratio which is equal to the test sample value divided by the value of a normal control serum.

The reactivity of MS patient sera with HTLV-I (mean ratio = 1.17) was significantly higher (Mann-Whitney U-test, p less than 0.003) than that of OND sera (mean ratio = 0.59) and of normal sera (mean ratio = 0.45, p less than 0.001). Reactivity of MS sera with HTLV-II (mean ratio = 1.63) did not differ significantly (p less than 0.08) from OND sera (mean ratio = 1.24). However, the MS group did differ significantly from the normals (mean ratio = 0.87, p less than 0.001). Serum reactivity with HTLV-III showed no significant differences between all groups. Serum antibody in three MS patients was shown to be significantly inhibited by HTLV-I, -II or -III antigen or specific antibody. No serum from OND or normal individuals are successfully competed in these assays. Competition was tested in two ways: by competition assays using extracts of virus producing cells, phytohaemagglutinin-stimulated normal human peripheral blood lymphocytes, and fetal calf serum; (2) by using HTLV-III type specific antibody and control normal antibody. Successful competition in either assay by more than 50% suggests specificity of antibody reaction.

Example 4

This example shows the amounts of antibody reactive with HTLV-I p24 in serial samples of CSF in sera obtained from Swedish MS patients during their disease, compared with concentrations of IgG and IgM in the same CSF and sera samples.

All samples were tested simultaneously in the same laboratory. Panel a shows patient 01-12, who showed onset of MS in September 1977 at the age of 34 years. The course of the disease was characterized by frequent, acute and short exacerbations, particularly during the study period from 1982-1984. The patient received no adrenocorticotrophin or steroid treatment. As can be seen in panel a, in this patient both the CSF and serum p24 antibody levels fluctuated markedly between 1982 and 1984. A peak of antibody in the CSF in February 1983 was accompanied by elevation of the serum antibody. Conversely the peaks of serum antibody observed in August 1983 and December 1984 were not accompanied by a rise in CSF antibodies. All antibody detected was of the IgG class; no IgM antibody was detected. The total IgG concentration in serum and CSF remained unchanged during the observation period; thus the increase in specific antibody could not be explained by polyclonal increases in IgG levels.

The second patient 20-01, has onset of MS in November 1976 at the age of 24 years. The course of the disease was characterized by exacerbations from its onset to 1983 with slow progression of the disease thereafter. The patient received two ACTH treatments in December 1980 and December 1981. As can be been in panel b, p24 reactivity was detected in both serum and CSF samples collected from 1977 to 1982; the two curves were parallel except from November 1981 on, when a rise in CSF antibody was accompanied by a decrease in serum antibody. Again, all antibody was IgG; no IgM antibody was detected. Fluctuation in antibody concentration did not reflect changes in CSF and serum IgG levels, which remained relatively constant.

In a third Swedish patient, 13-10, onset of MS occurred in March 1976 at the age of 11 years with exacerbations in 1979, 1980, April and September 1983, and April 1984 with complete recovery between exacerbations. The patient was treated with ACTH only during April 1983, one year before the exacerbations in April 1984. In this patient it is possible to follow the p24 antibody levels over several months before and during an acute clinical exacerbation in April 1984. A dramatic rise in CSF p24 antibody of the IgM was detected between September 14, 1983 and April 3, 1984, accompanied by a lesser but still significant rise in CSF IgG antibody to p24. During April 1984, the CSF IgG antibody decreased to its original value. The IgM CSF antibody levels remained high until April 13, 1984 after which they returned to levels observed before exacerbation. The IgM serum antibody fluctuated, whereas the IgG serum antibody remained at relatively high levels during the observation period. None of these changes could be explained by concomitant changes in CSF or serum IgG concentrations, which remained relatively stable during the observation period.

The assay for HTLV-I p24 antibodies used was the same as that described in Example 1. The immunoglobulin concentrations were determined by an electroimmunoassay described in The Scandinavian Journal of Clinical Laboratory Investigation Vol. 29, Supplement 124, 21-37 (1972) by Laurell. Serum samples were diluted for the ELISA assay to achieve approximately the same immunoglobulin concentration as found in the CSF samples. In order to compare the relative amounts of specific antibody. The immunoglobulin concentrations plotted are those used in the assay.

Example 5

This example demonstrates HTLV reactivity in sera from MS patients in Key West, Florida.

At the time of case assessment on December 1, 1983, 37 patients with MS were identified. Every patient but one was Caucasian. Among the patients were two pairs of siblings with MS and two MS patients with MS-afflicted siblings living elsewhere. The diagnosis of MS was based on the criteria of McDonald and Halliday.

Seventeen of the 37 MS patients were available for this study, 5 of whom were born in Key West; 5 had relocated to Key West at ages of 5 to 18 years and seven had relocated to Key West at ages 22 to 39 years. In 12 patients the onset of MS preceded the study from one to five years; in one patient by nine years and in the remaining patients, by 14, 16, 19 and 20 years. Three patients never received steroid therapy, 13 had received steroid or ACTH therapy, and one patient currently receives Immuran.

Paired samples of CSF and serum were available from nine of the 17 patients, and serum samples were available from the remaining 8 patients. A serum sample obtained 4 weeks later was available from 9 of the 17 patients. Seventeen close contacts of the patients (spouse, parent, or sibling) and 17 other individuals donated blood for this study.

Results can be seen in Table 2 below.

TABLE 2

HTLV-Reactivity of Sera from Key West Patients, Intimate Contacts and Hospital Workers

| Clinical Status | No. of Patients | No. of Sera Reacting With: HTLV-I p24 | Disrupted Virions of HTLV-I | Disrupted Virions of HTLV-II | Disrupted Virions of HTLV-III | No. of Positive Cases/Total |
|---|---|---|---|---|---|---|
| MS | 17 | 3 | 7 | 4 | 1 | 10†/17 |
| Contacts | 17 | 1 | 0 | 0 | 1 | 2††/17 |
| Hospital Workers | 17 | 0 | 0 | 0 | 0 | 0/17 |

† Serum of one patient reacted with all three types of HTLV; serum of another patient reacted with types I and II, and serum of one patient reacted only with HTLV-I p24.

†† One case was a homosexual brother of an MS patient.

As can be seen in Table 2, above, seven serum samples from MS were reactive with HTLV-I, four with HTLV-II, and one with HTLV-III; three sera reacted in addition with HTLV-I p24. Serum of one patient reacted with all three HTLV types whereas the serum of one patient reacted with HTLV-I and -II. A total of ten of the 17 MS sera were reacted with an HTLV antigen at least in one test in time. Of the 17 donors who had intimate contact with the MS patients, sera from two showed reactivity with HTLV-III. One of the sera, obtained from a homosexual brother of one of the MS patients was HTLV-III sera positive. His serum reactivity might reflect true exposure to the HTLV-III agent of AIDS rather than a cross reactivity. The second was a male not known to be in any HTLV high-risk group. None of the sera from 17 hospital workers where most of the MS cases were treated showed reactivity with HTLV-I, -II, or -III.

Of nine CSF samples available from these MS patients, only one sample was reactive with HTLV antigens, specifically, with HTLV-I p24. Of the eight negative samples, seven were obtained from patients whose sera showed the presence of antibodies reactive with HTLV antigens.

The reactivity was determined by ELISA as described above in Example 1. The results were all confirmed by competition with either antigens of, or specific antibodies to HTLV-I, -II, and -III. All samples were tested simultaneously in the same laboratory.

Example 6

This example shows that HTLV-IV infected cells can be detected using in situ RNA hybridization to an HTLV-I probe.

In situ hybridization was used to examine CSF cells in culture obtained from five Key West MS patients, three Swedish MS patients and two healthy Swedish subjects for the presence of HTLV specific RNA by methods described in Harper, M.E., et al. Proceedings of the National Academy of Science (USA) (1986) in press. Three of the CSF cultures derived from Key West MS patients with duration of disease 3, 4 and 21 years, and one CSF culture derived from a Swedish MS case with duration of disease of one month studied to date showed the presence of labeled cells following hybridization under low stringency conditions (45°) with the HTLV-I 3' RNA probe which predominantly contains LTR sequences. These positive cells exhibited an average of 50 grains per cell and were observed at a frequency of less than .01% of the 5 x $10^5$ cells generally examined. In contrast when the same probe was hybridized under identical conditions to cultured CSF cells from two normal individuals no labeled cells were revealed. Similarly, normal peripheral blood cells, bone marrow and other cell types showed no labeled cells when they were examined by this method.

Hybridization of an HTLV-I probe specific for the p24 gag coding region was used to label cells from CSF of two Key West MS patients; again rare labeled cells were found under low stringency conditions.

Thus in total, four MS CSF cultures tested contained cells expressing RNA homologous to HTLV-I. In several experiments in which hybridization with MS CSF cells from one Key West patient was carried out under standard stringent conditions (50°C), no labeled cells were observed. Although difficult to quantitate because of the low number of positive cells observed after hybridization at 45°C, the lack of detectable label at high stringency conditions suggests that the RNA detected shares only partial homology with the HTLV-I genome. No hybridization was detected in the same MS CSF cultures using an HTLV-III 3' RNA probe, under reduced stringency indicating a lack of RNA homologous at this level to this virus in the cultured cells.

The labeling procedure was conducted as follows: freshly isolated CSF cells were co-cultured in cluster plates (Costar) with autologous gamma irradiated peripheral blood mononuclear cells in complete medium containing purified phytohaemmoglutinin P, at 1 ug/ml, obtained from (Burroughs-Wellcome) for three days. Cell concentrations were adjusted to 2 x $10^5$ cells/ml in complete growth media with 50 ng/ml recombinant interleukin-2 (obtained from Sandoz Ltd, Vienna, Austria). Cells were maintained in logarithmic growth by periodic supplementation with fresh media containing interleukin-2 and by stimulation with phytohaemmoglutinin P. All cell lines were maintained in the same lab before hybridization.

*In situ* hybridization was carried out using $^{35}S$-labeled RNA probes specific for the 3' region of HTLV-I. The RNA probes were transcribed by SP6 polymerase (Promega Biotech) from a full length genomic clone of HTLV-I and were on the average 1-2 kilobases long. The probes were hybridized at 1-2 x $10^8$ dpm/ml under relaxed conditions (45°C) in a hybridization mixture containing 50% formamide, 2 x SSC, 10 mM DTT (dithiotreitol) and 1 mg/ml sheared salmon sperm DNA. (1xSSC consists of 0.15 M NaCl, 0.015 M Na-citrate.) After hybridization, RNase A (100 ug/ml) and RNase $T_1$ (1 ug/ml) were added to remove any non-hybridized (single stranded) RNA. Cells were autoradiographed for 4-8 days with NTB2 nuclear track emulsion (Eastman) diluted 1:1. All cell lines were hybridized in the same conditions in the same laboratory and cell were examined using a double-blind code.

The foregoing examples are not intended to limit the scope of the invention, but are merely presented as exemplification of the specification. The invention is limited solely by the claims appended below.

CLAIMS

1. A diagnostic method to identify human patients suffering from MS comprising: testing for the presence of HTLV-IV.

2. The diagnostic method of claim 1 comprising:

providing a body fluid of a human patient, said fluid selected from a group consisting of cerebrospinal fluid and serum;

contacting said body fluid with HTLV-IV proteins under conditions where antibody-antigen complexes form and are stable;

determining the presence or absence of antibody-antigen complexes.

3. The diagnostic method of claim 1 comprising:

providing a body fluid of a human patient said fluid selected from the group consisting of cerebrospinal fluid and serum;

contacting said body fluid with antibodies immunoreactive with HTLV-IV;

determining the presence or absence of antibody-antigen complexes.

4. The diagnostic method of claim 1 comprising:

providing a body fluid of a human patient, said fluid selected from a group consisting of cerebrospinal fluid and serum;

contacting said body fluid with HTLV-I proteins under conditions where antibody-antigen complexes form and are stable;

determining the presence or absence of antibody-antigen complexes.

5. The diagnostic method of claim 1 comprising:

providing at least two samples of a body fluid of a human patient said fluid selected from the group consisting of cerebrospinal fluid and serum;

contacting a first sample of said body fluid with disrupted HTLV-I virions;

further contacting said first sample and a second sample of said body fluid with HTLV-I p24 <u>gag</u> protein; and

quantitating the antibody-p24 gag protein complexes in the two samples to determine if the amount of antibody p24 gag protein complexes in the first sample is at least about 15% of the amount of the second sample.

6. A method of identifying a cell infected with HTLV-IV virus comprising:

providing a sample of a body specimen of a human who has presented symptoms of multiple sclerosis, said sample containing at least $10^4$ cells;

contacting said sample with a nucleic acid probe having HTLV-IV homologous sequences;

treating said sample to remove unhybridized nucleic acid probe;

detecting hybridized nucleic acid probe, the localizations of said hybridized nucleic acid probe identifying the cells infected with HTLV-IV virus.

7. The method of claim 6 wherein hybridization is performed at high stringency conditions.

8. The method of claim 7 wherein the nucleic acid probe comprises RNA transcribed from HTLV-IV sequences.

9. The method of claim 6 wherein hybridization is performed at low stringency conditions.

10. The method of claim 9 wherein the nucleic acid probe comprises RNA transcribed from HTLV-I sequences.

11. The method of claim 10 wherein the nucleic acid probe comprises RNA transcribed from HTLV-I p24 gag or LTR sequences.

12. The method of claim 10 further comprising:

providing a second sample of a body specimen of a human who has presented symptoms of multiple sclerosis, containing at least $10^4$ cells;

contacting the second sample of said body specimen with said nucleic acid probe under high stringency conditions;

treating said sample to remove unhybridized nucleic acid probe;

detecting said hybridized nucleic acid probe with the localizations of said hybridized nucleic acid probe on said first sample identifying the cells infected with HTLV-IV when said second sample contains significantly less hybridized nucleic acid probe.

13. The method of identifying a cell infected with HTLV-IV virus comprising:

providing a body specimen of a human who has presented symptoms of multiple sclerosis and who has serum containing antibodies immunoreactive with p24 gag protein of HTLV-I, immunoreactivity of said serum antibodies with p24 gag protein of HTLV-I cannot be completely competed by disrupted virions of HTLV-I, said body specimen containing at least $10^4$ cells;

contacting said body specimen with an antibody immunoreactive with p24 gag protein of HTLV-I, under conditions where antigen-antibody complexes can form and are stable, said antibody bearing a means of detection; and

detecting antigen-antibody complexes formed, the localization of antigen-antibody complexes identifying the cells infected with HTLV-IV.

14. A method of identifying HTLV-IV virus comprising:

providing a virus and extracting RNA from said virus; and hybridizing viral nucleic acid with a probe comprising HTLV-IV homologous sequences at condition of high stringency and selecting HTLV-IV RNA that hybridizes to said probe.

15. The method of claim 14 wherein said HTLV-IV homologous sequences comprise HTLV-IV sequences.

16. The method of claim 14 wherein said HTLV-IV homologous sequences comprise HTLV-I sequences, and hybridization is performed at conditions of low and high stringency, and HTLV-IV RNA is selected that hybridizes to a significantly greater extent at low stringency than at high stringency.

17. The method of claim 16 wherein the HTLV-I sequences are selected from the group consisting of p24 gag gene and LTR sequences.

18. A vaccine for immunizing humans against multiple sclerosis comprising protein of HTLV-IV.

19. The vaccine of claim 18 wherein the proteins of HTLV-IV are provided by a preparation of attenuated virions.

20. The vaccine of claim 18 wherein the proteins of HTLV-IV are provided by whole virus which is a nonpathogenic variant of HTLV-IV.

FIG. 1

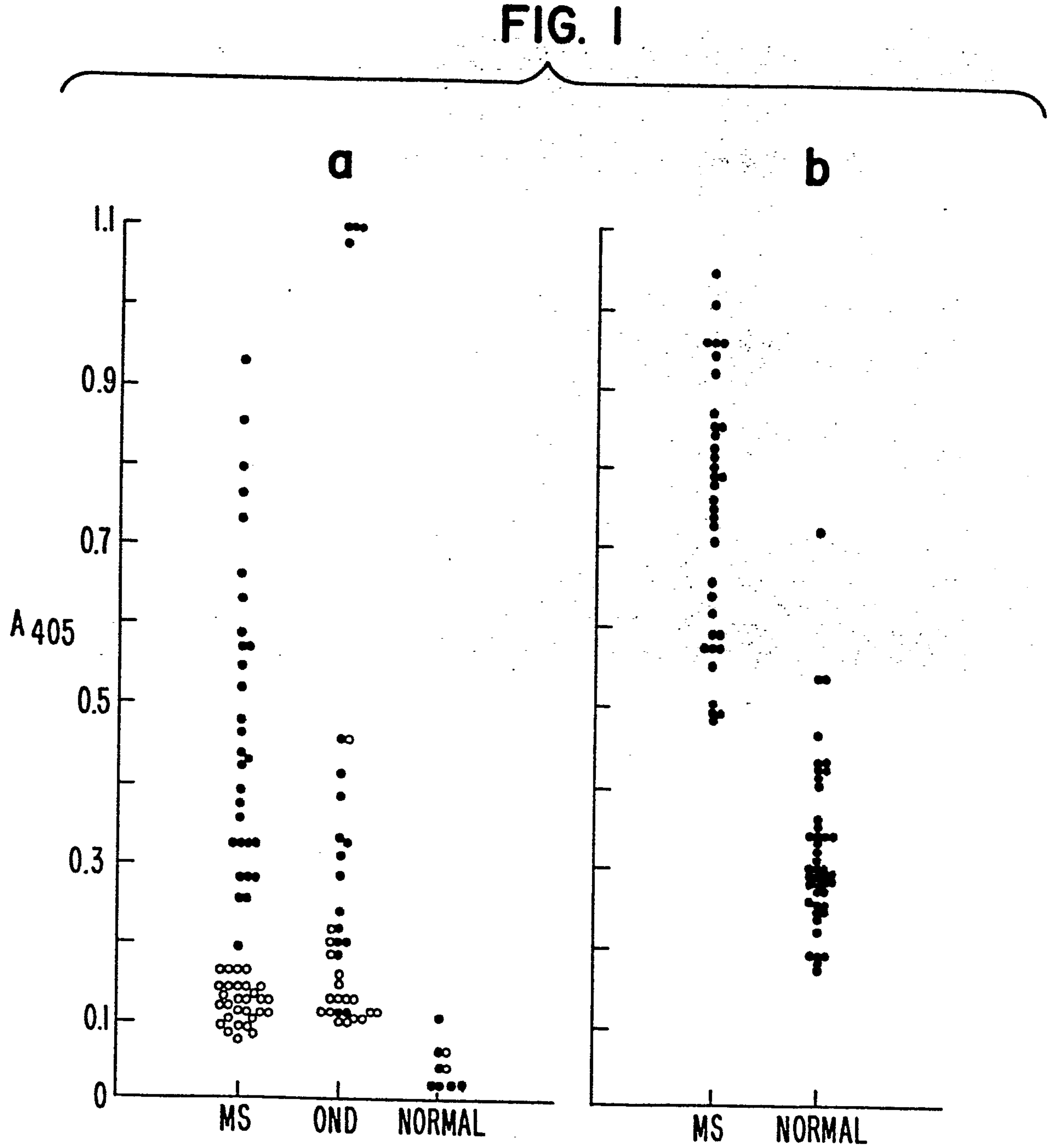

FIG. 2a

FIG. 2b

FIG. 2c

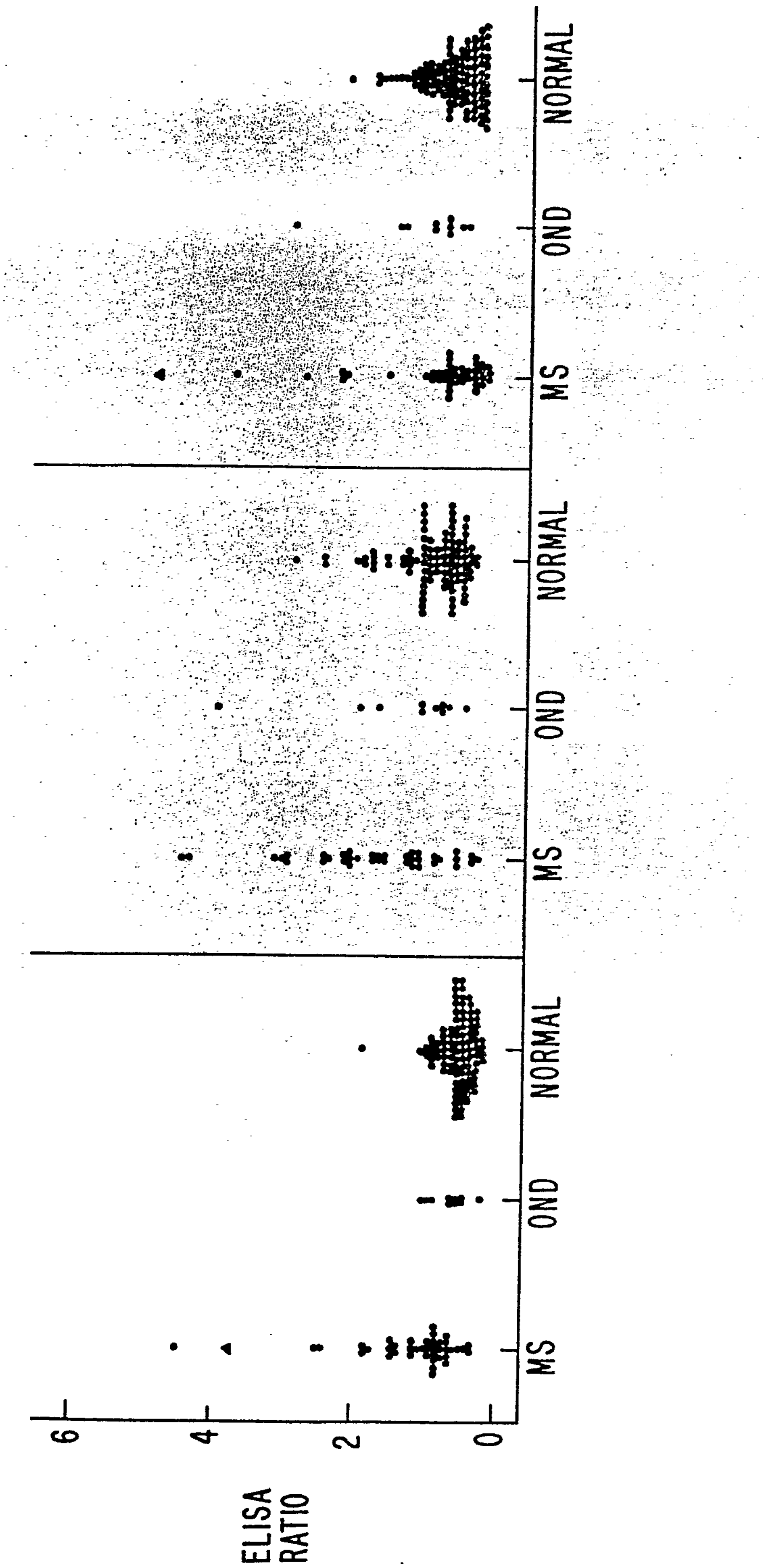

FIG. 3a

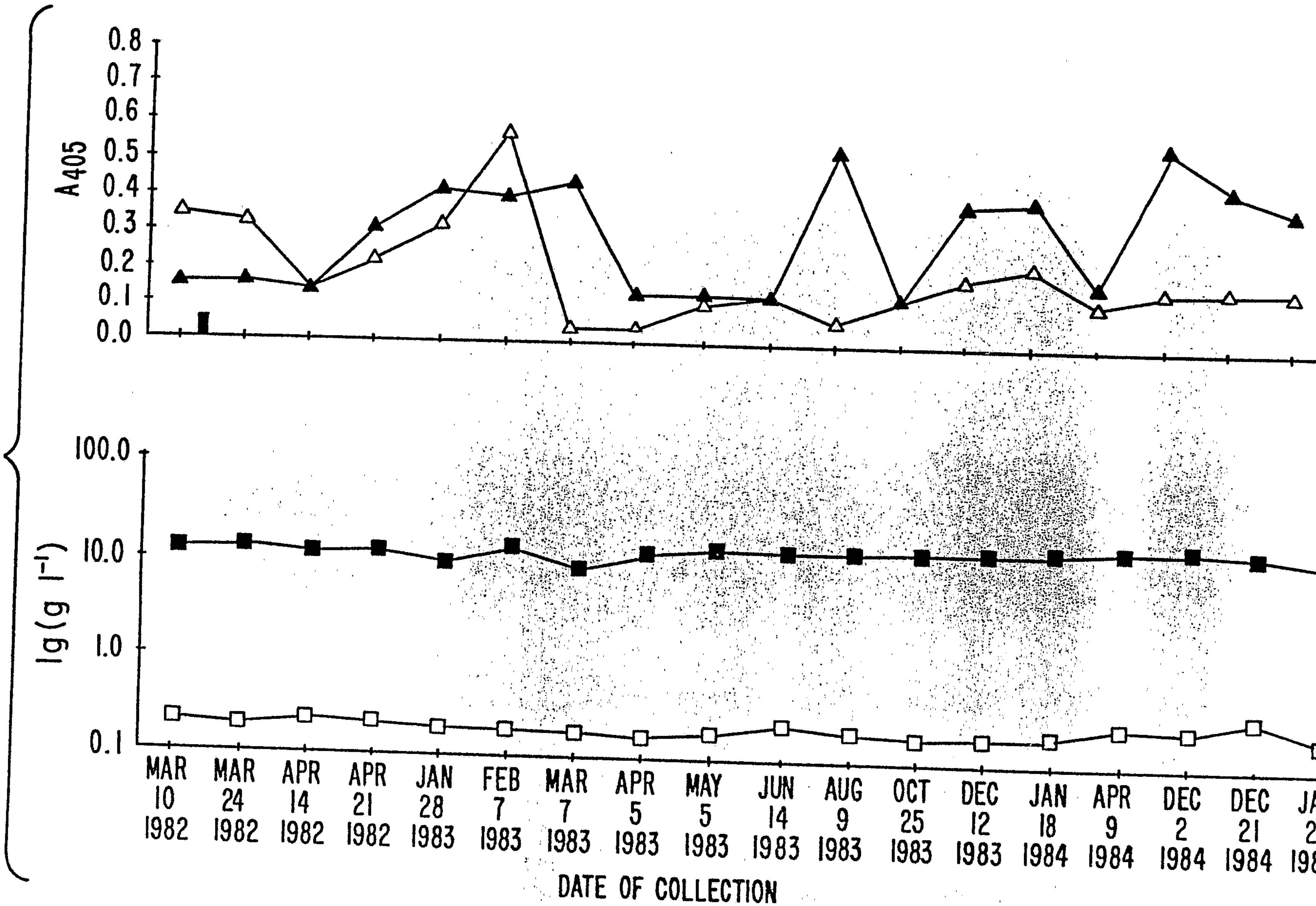

A405
0.8
0.7
0.6
0.5
0.4
0.3
0.2
0.1
0.0
lg(g l⁻¹)
100.0
10.0
1.0
0.1
MAR 10 1982
MAR 24 1982
APR 14 1982
APR 21 1982
JAN 28 1983
FEB 7 1983
MAR 7 1983
APR 5 1983
MAY 5 1983
JUN 14 1983
AUG 9 1983
OCT 25 1983
DEC 12 1983
JAN 18 1984
APR 9 1984
DEC 2 1984
DEC 21 1984
JAN 23 1985
DATE OF COLLECTION

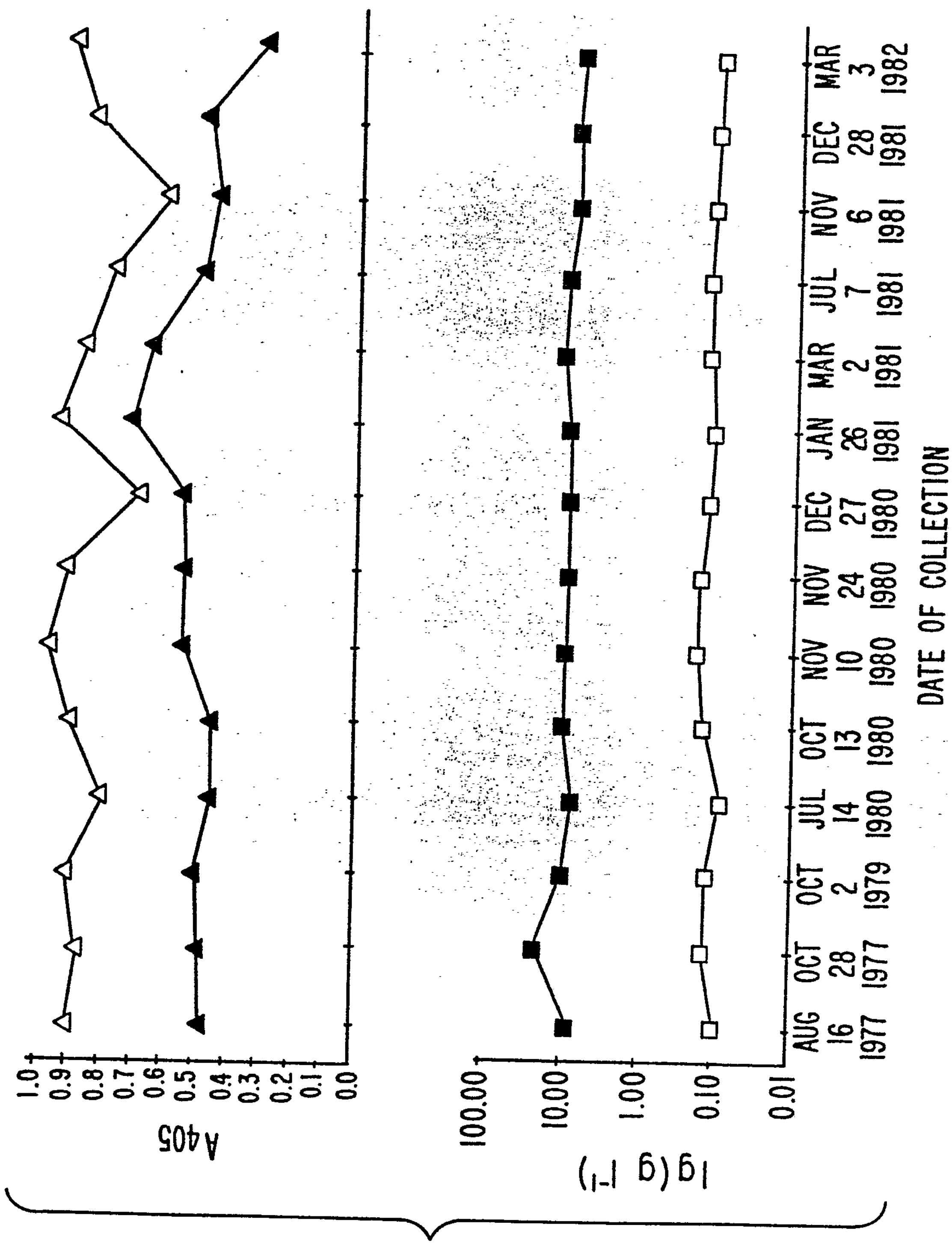
A405
1.0
0.9
0.8
0.7
0.6
0.5
0.4
0.3
0.2
0.1
0.0
Ig (g l-1)
100.00
10.00
1.00
0.10
0.01
AUG 16 1977
OCT 28 1977
OCT 2 1979
JUL 14 1980
OCT 13 1980
NOV 10 1980
NOV 24 1980
DEC 27 1980
JAN 26 1981
MAR 2 1981
JUL 7 1981
NOV 6 1981
DEC 28 1981
MAR 3 1982
DATE OF COLLECTION

FIG. 3b

FIG. 3c

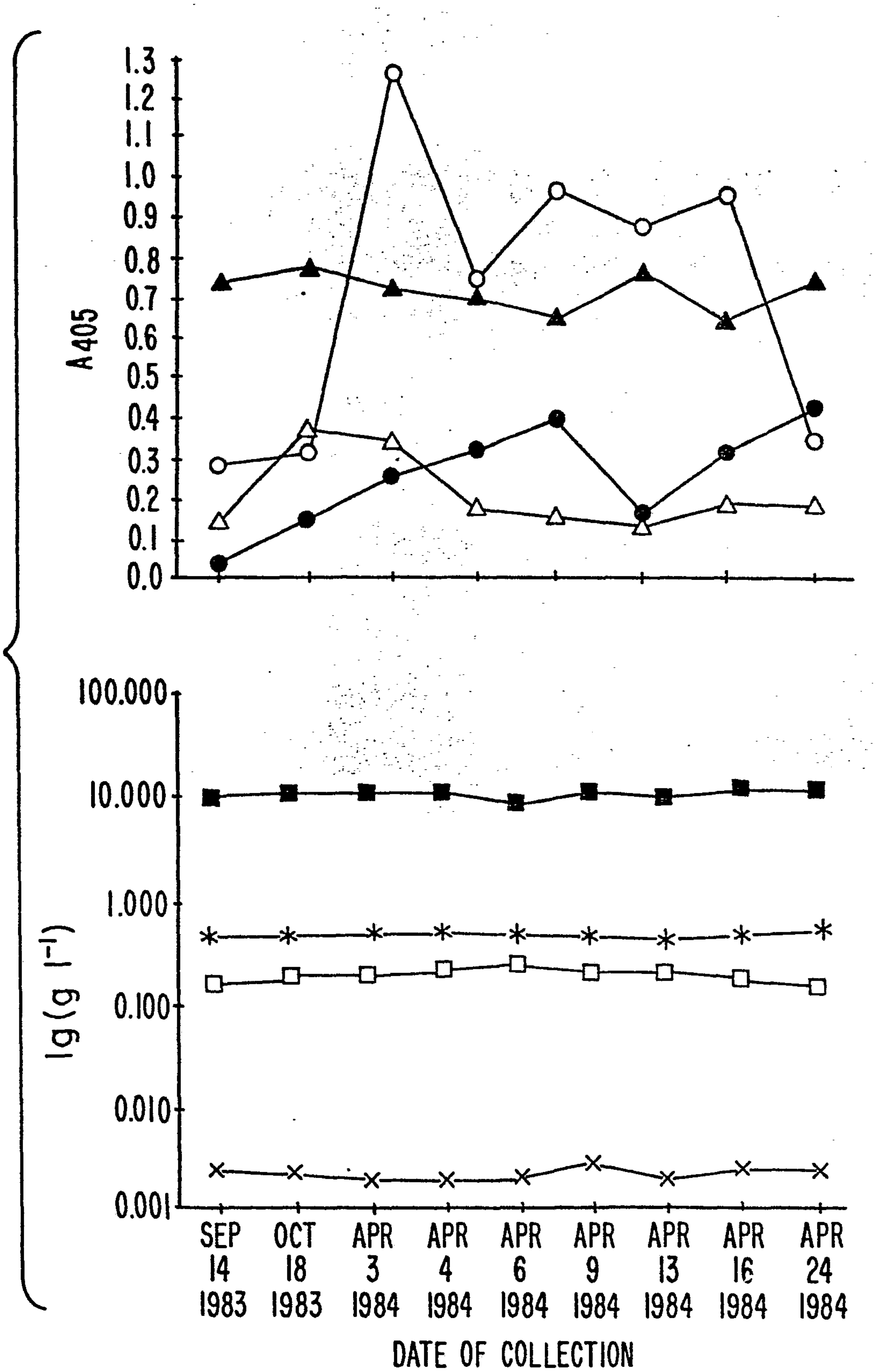
A405
1.3
1.2
1.1
1.0
0.9
0.8
0.7
0.6
0.5
0.4
0.3
0.2
0.1
0.0
lg(g l⁻¹)
100.000
10.000
1.000
0.100
0.010
0.001
SEP 14 1983
OCT 18 1983
APR 3 1984
APR 4 1984
APR 6 1984
APR 9 1984
APR 13 1984
APR 16 1984
APR 24 1984
DATE OF COLLECTION

FIG. 4 a

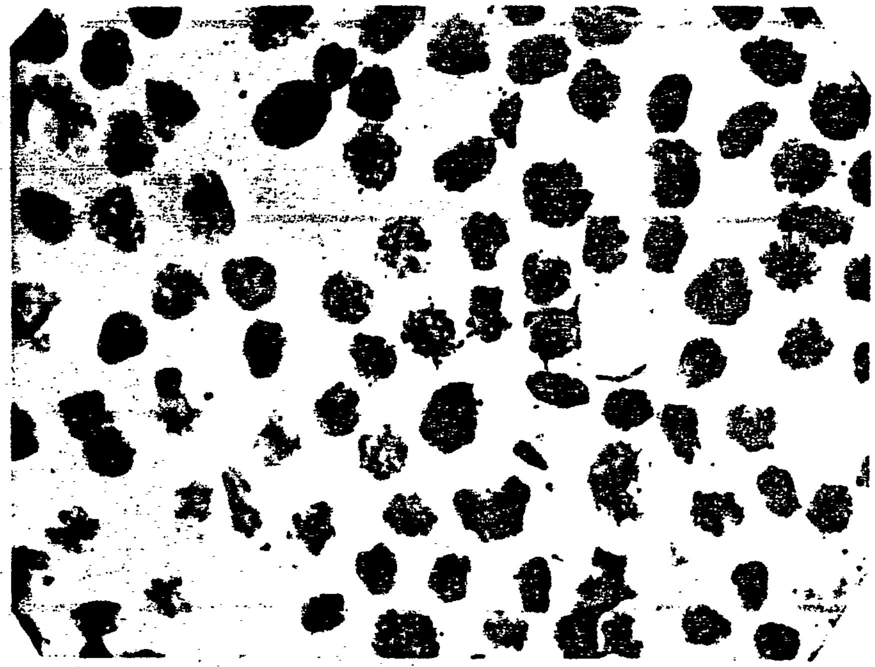

FIG. 4 b